# EUROPEAN PATENT SPECIFICATION

(11) **EP 4 142 471 B1**
(45) Date of publication and mention of the grant of the patent: **18.11.2026**
(21) Application number: 21797775.0
(22) Date of filing: 29.04.2021
(51) Int. Cl.: A61L 2/20, A61L 2/202, A23B 7/14, A01G 7/00, A01N 59/00, A23B 7/144

(54) **SYSTEM AND METHOD FOR TREATING HARVESTED PLANT MATERIAL WITH OZONE**
SYSTEM UND VERFAHREN ZUR BEHANDLUNG VON GEERNTETEM PFLANZENMATERIAL MIT OZON
SYSTÈME ET PROCÉDÉ POUR TRAITER UN MATÉRIAU VÉGÉTAL RÉCOLTÉ AVEC DE L'OZONE

(30) Priority: 30.04.2020 US 202063018418 P; 03.06.2020 US 202063034222 P; 20.08.2020 US 202063068038 P
(43) Date of publication of application: 08.03.2023
(73) Proprietor: Willowpure, LLC, Denver, CO 80216 (US)
(72) Inventor: ELLSWORTH, Jill, Lynn, Aurora, CO 80012 (US); ELLSWORTH, Jason, Robert, Aurora, CO 80012 (US); OTTMAN, Mark, Steven, Denver, CO 80216 (US); SINGLETON, Brian, Dunn, Denver, CO 80220 (US); KULE, Joshua, Adam, Denver, CO 80205 (US); ALVAREZ, Adrian, Leon, San Diego, CA 92101 (US)
(74) Representative: Pohlman, Sandra M.
(86) International application number: PCT/US2021/029832
(87) International publication number: WO 2021/222526

(56) References cited:
- US-A1- 2010 028 510
- US-A1- 2010 119 606
- US-A1- 2018 207 307
- US-A1- 2019 124 865
- US-A1- 2019 314 535
- US-A1- 2020 068 899
- US-B1- 6 171 625
- US-B2- 7 939 016

## Description

### Field of the Disclosure

This disclosure relates to reducing contamination of harvested plant material.

### Background

Harvested plants can contain any of a variety of contaminants such as fungus (including yeast and mold), bacteria, viruses and pesticides. Harvested plants must often comply with regulations regarding permissible levels of contaminants so that they are safe for human use or consumption. For example, cannabis and cannabis-based products intended for human consumption are often tested before sale for microbial contaminants, residual solvents and pesticides.

Solutions are needed to substantially reduce contamination of plant material post-harvest. Such solutions would ideally include maintaining the quality and attributes of the plant material that are important to consumers. Included among these are the structure, appearance, and THC, CBD and terpene content in cannabis plant material, for example.

US 2018/0207307 A1 and US 2019/0314535 A1 disclose methods, apparatus, and systems for disinfecting and sterilizing medical equipment using ozone. US 2020/0068899 A1 discloses the ozone treatment of plants to reduce or eliminate pesticides, mold, yeast, or other pathogens. US 2010/0119606 A1 discloses a rotatable drum for preparing extracts of natural products, such as plant material, by extraction with hot gas.

### Summary

The present disclosure relates to systems and methods for treating harvested plant material with ozone, including systems and methods for tumbling the harvested plant material in a rotating drum while exposing the material to ozone. Tumbling the material can result in exposing a larger surface area of the material to ozone treatment, and producing a more homogenous treated product, compared to arrangements in which the material remains in a static position during treatment. The systems and methods can be used to treat cannabis plant material, for example, to reduce the amount of pathogens or pesticides in the material so that the product meets regulatory and other safety requirements for use by a consumer.

The present invention is set out in the appended set of claims. The invention provides a method for treating harvested plant material, which comprises tumbling the harvested plant material within a rotating vessel while exposing the material to ozone, wherein the plant material comprises at least a portion of a cannabis plant. More embodiments and features are included in the detailed description that follows, and will be readily apparent to those skilled in the art from the description or recognized by practicing the embodiments as described in the description, including in the figures and claims.

### Brief Description of the Drawings

The accompanying figures constitute a part of this disclosure. The figures serve to provide a further understanding of certain exemplary embodiments.
FIG. 1A is a perspective view of an exemplary drum positioned horizontally to the ground.
FIG. 1B is a side view of the drum in FIG. 1A.
FIG**.** 1C is a cross-sectional view of the drum in FIG. 1A.
FIG. 1D is a side view of an exemplary drum positioned at an angle to the ground.
FIG. 2 is a perspective view of a system of the disclosure, with a drum positioned outside of a safety enclosure.
FIG. 3A is a perspective view of the system of the disclosure, with a drum positioned within a safety enclosure.
FIG. 3B is an enhanced view of a bracket and roller illustrated in FIG. 3A.
FIG. 4A is a top view of the system in FIG. 3A.
FIG. 4B is a side view of the system in FIG. 3A.
FIG. 5 is a front view of an exemplary safety enclosure.
FIG. 6 is a perspective view of an exemplary destruct fan assembly.
FIG. 7 is a P&ID schematic of an embodiment of a system of the disclosure.
FIG. 8 is a simplified diagram of a distributed computing system in which aspects of the present disclosure may be practiced.
FIG. 9 illustrates one example of a suitable operating environment in which aspects of the present disclosure may be implemented.
**FIG.** 10 illustrates an additional example of a system of the disclosure discussed in Example 2.
FIG. 11 illustrates an additional example of a system of the disclosure discussed in Example 3.

### Detailed Description

In some embodiment of the method of the invention, the harvested plant material is placed in the vessel of a system for treating harvested plant material with ozone, comprising:
an ozone generator; and
a vessel in fluid communication with the ozone generator;
wherein the vessel comprises an interior volume for containing harvested plant material and is configured to rotate about an axis.

The term "ozone" as used herein refers to gaseous ozone. Ozone occurs naturally at low levels, but can be produced using any of a variety of techniques, such as the corona discharge method, narrow-band UV light method, the cold plasma method, and electrolytic ozone generation. The ozone generator in the system of the disclosure can be any appropriate device for producing ozone gas, such as but not limited to a corona discharge ozone generator. The ozone may be included within a feed gas that comprises additional components such as oxygen, nitrogen, water vapor, argon and/or carbon dioxide.

A vessel "in fluid communication" with the ozone generator refers to a vessel that is configured such that it can receive a flow of ozone gas produced by the ozone generator. In some embodiments, other system components may be positioned between the ozone generator and vessel, such as valves, bubblers or other equipment, such that the ozone gas would pass through such components before reaching the vessel. Possible materials of construction for the vessel include, for example, plastic, aluminum, an aluminum alloy, anodized aluminum or anodized aluminum alloy, and stainless steel.

The vessel comprises an interior volume for containing harvested plant material. The vessel is configured such that ozone gas received from the ozone generator can flow into the interior volume of the vessel and contact harvested plant material disposed therein. The vessel is configured to rotate about an axis so that it may tumble harvested plant material being exposed to ozone.

The terms "tumble" and "tumbling" refer to the physical movement, relative to the drum, of all or a portion of harvested plant material within the vessel in response to the vessel's rotation. Tumbling can include, but does not require, all or a portion of the harvested plant material becoming airborne within the tumbler when the vessel is rotating. Tumbling can include the shifting of some portion of plant material relative to another portion of plant material in the vessel. The plant material may tumble in the vessel simply due to the force of gravity as the vessel rotates, but the system of the disclosure does not exclude the possibility of additional components or moving parts to assist in physically moving the plant material. Tumbling can also result in the mixing of plant material such that the material becomes more homogenous.

In some embodiments, the vessel is a rotatable drum having a length extending between two opposite ends and having a cross-section along its length, the length and cross-section together defining the interior volume of the drum. Such a drum could comprise a circular cross-section along at least a portion of its length. For example, the drum could be cylindrical in shape. In other embodiments, the drum could comprise a circular cross-section that varies in radius along at least a portion of the length of the drum. The drum may have any other appropriate shape or cross-section such as spherical, elliptical, oval or oblong shapes or cross-sections, or shapes or cross-sections comprising angles and vertices in addition to or as an alternative to curves.

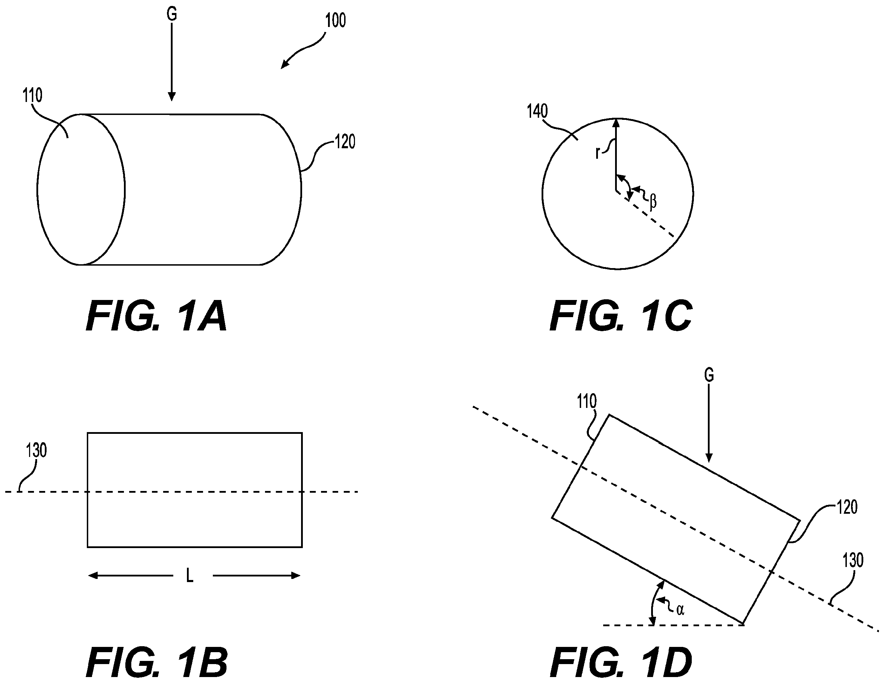

FIG.1A is a perspective view of a cylindrical drum 100. FIG. 1B is a side view of such a drum, and FIG. 1C shows a view of the cross-section of the drum. The drum has a length L extending between two opposite ends 110 and 120 and a cross section 140 having a radius r, these dimensions defining the drum's interior volume. The interior of the vessel, such as the cylindrical drum shown in FIGs. 1A-1D, may be any appropriate volume, such as from 1.89 L to 11,356.24 L, for example from from 113.56 L to 1892.71 L.

The drum illustrated in FIGs. 1A-1D is configured to rotate about longitudinal axis 130 that is located in the center of the cross-section 140 and extends in a direction normal to the cross-section. FIG. 1A illustrates the drum positioned horizontally such that the axis of rotation is perpendicular to the direction of gravity G. The drum could alternatively be positioned at an angle α such that its axis of rotation is not perpendicular to the direction of gravity, such as shown in FIG. 1D, where 110 is the top of the drum (which may optionally include a lid), 120 is the base of the drum and 130 is the axis. The angle α to the direction of gravity may be selected as appropriate to adjust the desired tumbling conditions. For example, the angle α may be 5° or less, 10° or less, 15° or less, 20° or less, 30° or less, 40° or less, or 45° or less, and any angle between 45° and 90°.

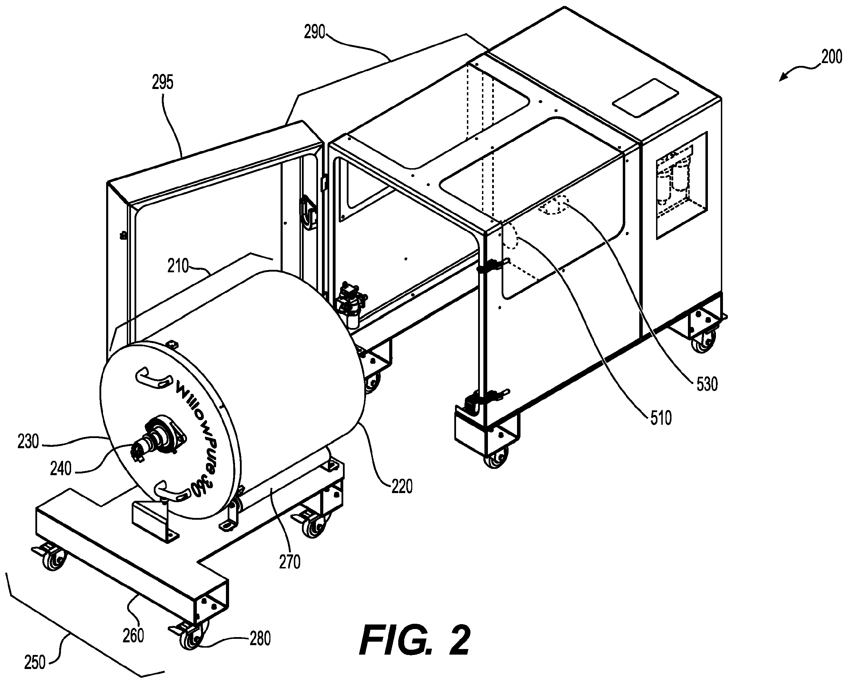

The vessel can comprise an inlet for gas to enter the vessel and an outlet for gas to exit the vessel. The vessel may be in the form of a main body and a lid, and in some embodiments may include the gas inlet and/or outlet positioned on the lid. FIG. 2 is a perspective view of a system of the disclosure 200, with a drum 210 positioned outside of a safety enclosure 290, which includes open door 295. The drum is in the form of a main body 220 and lid 230 and is an ozone chamber designed to contain the gas within its interior volume.

The lid comprises a fixture 240 that comprises an inlet and outlet, each of which may comprise a valve. The inlet and outlet may connect to appropriate tubing to accommodate the flow of gas into and out of the vessel, respectively. For example, the inlet may connect to an ozone delivery tube through which fresh ozone gas enters the vessel. The end of the ozone delivery tube through which the ozone gas exits into the vessel may terminate at any appropriate location within the vessel. For example, the end of the ozone delivery tube may terminate inside the vessel near the vessel lid. Alternatively, the ozone delivery tube may extend further into the interior of the vessel, such as near the center of the vessel. With the end of the ozone delivery tube positioned near the interior center of the vessel, fresh ozone gas could potentially be dispersed more evenly throughout.

The system of the disclosure may further comprise a cart positioned under the vessel to support its weight, such as cart 250 shown in Fig. 2. Such a cart can comprise a platform (such as platform 260) and two or more rollers (270) mounted on the platform in contact with and parallel to the length of the vessel to provide freedom for the vessel's rotation. Fig. 2 illustrates one of the rollers 270, with the second roller 270 positioned on the other side the platform that is not visible. The cart may further comprise wheels (such as wheels 280) to facilitate movement of the platform along the ground.

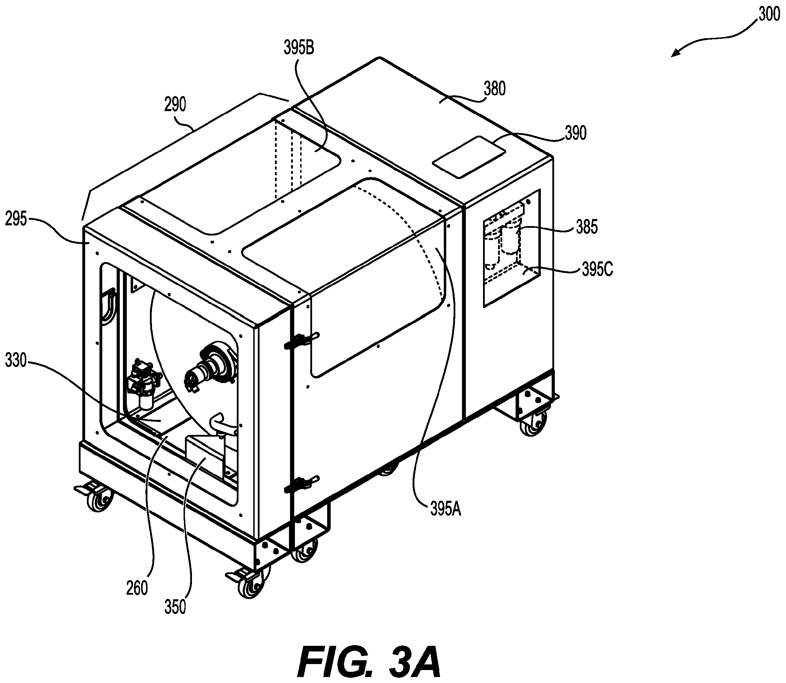

The system of the disclosure may comprise a safety enclosure in which the vessel can be contained, wherein the enclosure serves as a physical barrier between the vessel and outside environment. FIG. 2 illustrates an exemplary safety enclosure 290 comprising a door 295 that is opened to accommodate placement of the vessel. FIG. 3A illustrates a system 300 with the vessel placed inside the safety enclosure 290, with the door 295 of the safety enclosure closed.

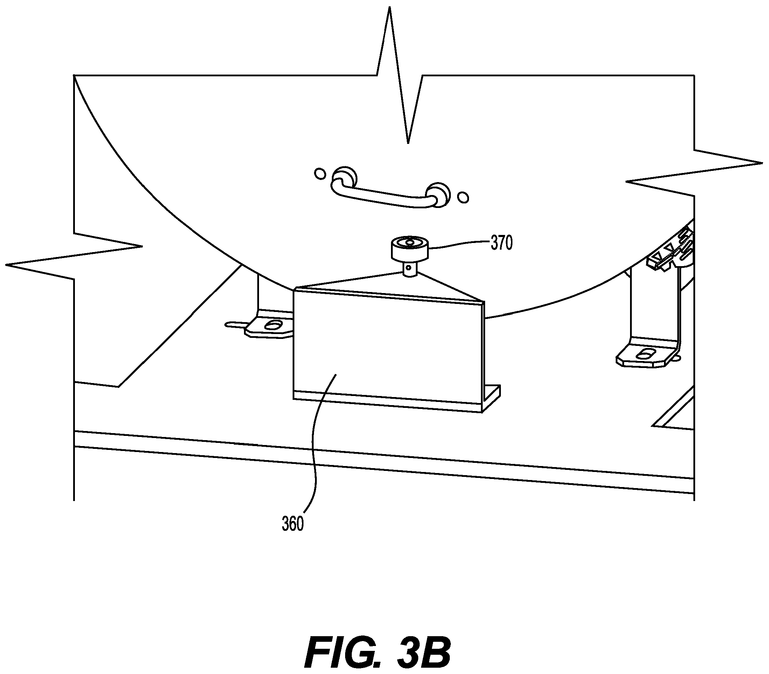

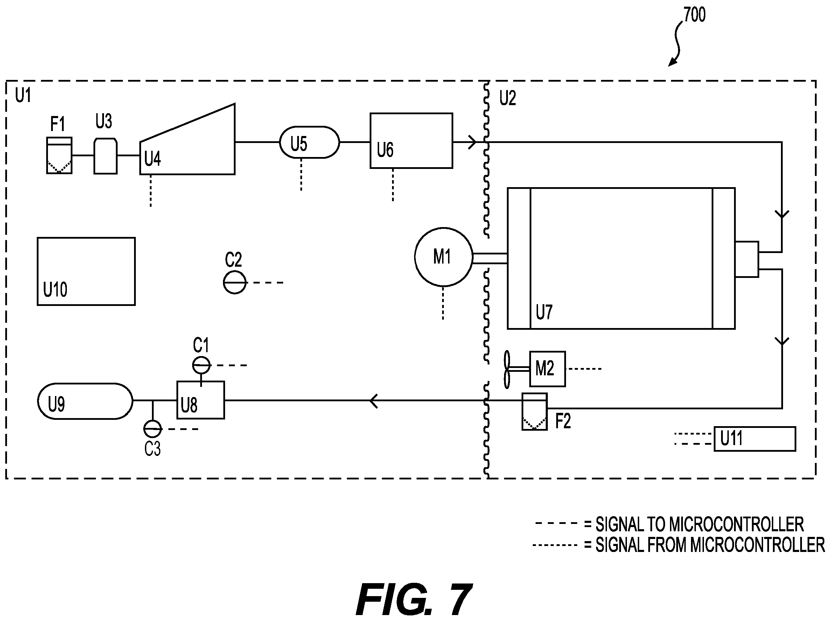

As can be seen in FIG. 3A, the safety enclosure comprises a floor 330, and at least a portion of the floor comprises the platform of the cart 260 positioned under the vessel. In this design, the floor of the enclosure is slotted to fit the cart such that the cart can be conveniently attached to and detached from the enclosure. A bracket and roller assembly 350 assists in maintaining the position of the vessel. FIG. 3B provides an enhanced view of the bracket 360 and roller 370 illustrated in FIG. 3A. The drum is visible through windows 395A and 395B. Two cylinders 385 shown in dotted lines behind window 395C (and also shown in FIG. 2) are components F1 and U3 illustrated in the schematic of FIG. 7.

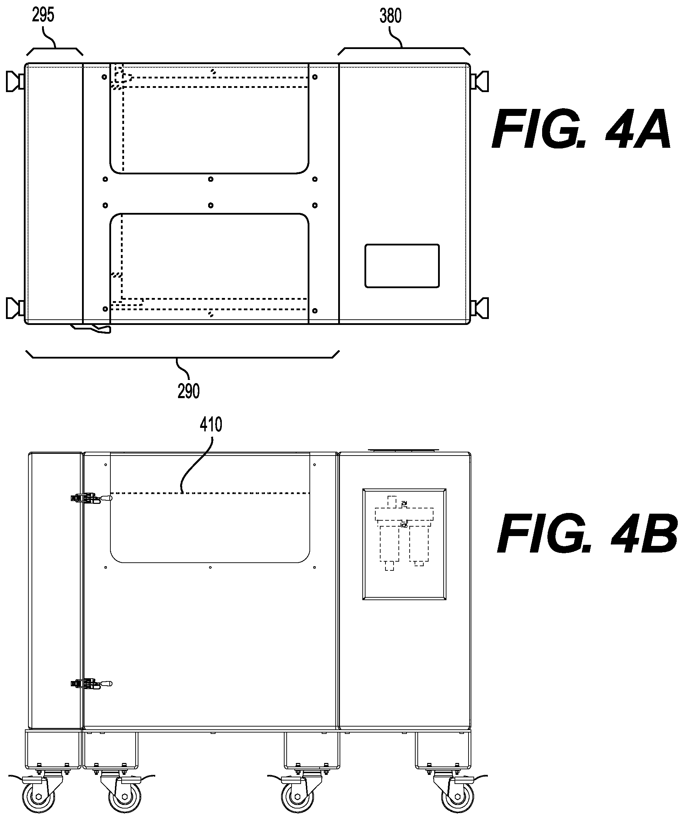

FIGs. 4A and 4B are top and side views, respectively, of the system in FIG. 3A. FIGs. 3A, 4A and 4B illustrate a safety enclosure that optionally includes windows 395A and 395B for viewing operation of the vessel inside the safety enclosure. The dashed line 410 in FIG. 4B represents the surface of the drum viewable through window 395A.

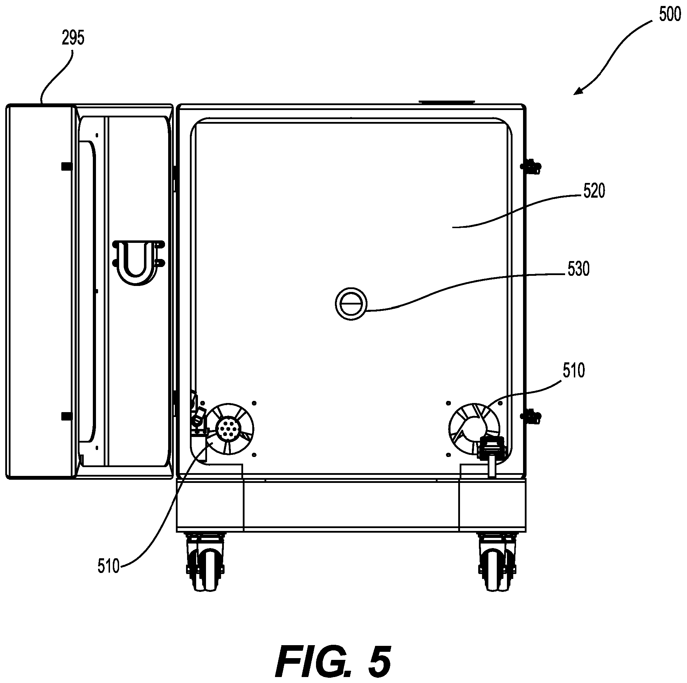

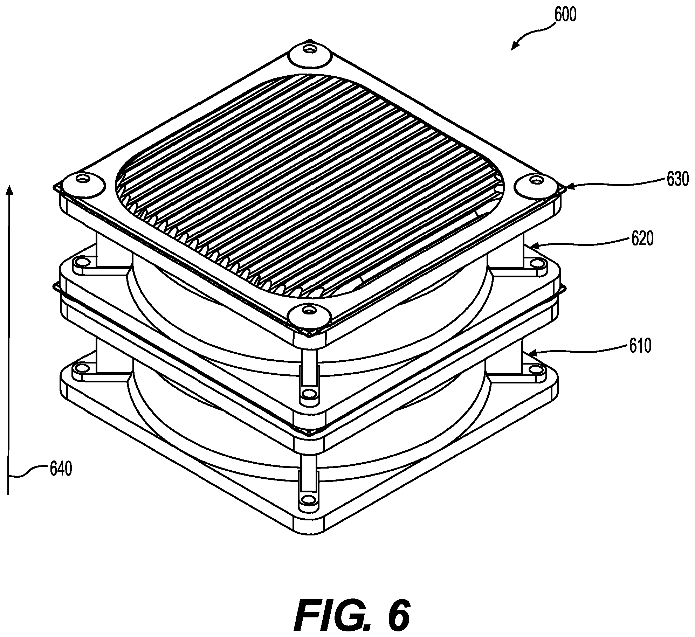

FIG. 5 is a front view of a safety enclosure 500, with an open door 295, that does not yet include the vessel positioned inside. In some embodiments, the safety enclosure is fitted with one or more fans configured to withdraw gas from the enclosure and thereby provide negative pressure within the enclosure. FIG. 5 illustrates two such fans 510. One or more of the fans can be mounted to a container that comprises catalyst media to scrub withdrawn gas of ozone before the withdrawn gas is released into the atmosphere. FIG. 6 is a perspective view of an exemplary fan assembly 600. The assembly includes high flow fan 610, carulite frame 620 filled with carulite and fan cover 630. Direction of air flow is illustrated by direction arrow 640. A fan 510 in FIG. 2 is also shown as viewed through a window of system 200.

The system may further comprise a means for rotating the vessel about its axis. As one example, the means for rotating the vessel about its axis can be a motor connected to the vessel by a shaft via a female coupler on the base of the vessel. The means for rotating the vessel about its axis could alternatively, or in addition, be a motorized roller in contact with the vessel. For example, one or more rollers, such as roller 270, could be rotated, such as with the use of a motor, to in turn rotate the vessel.

Safety enclosure 500 illustrates an exemplary design for an enclosed vessel to engage with a means for rotating the vessel about its axis. The enclosure includes a back wall 520 having a passage through which the vessel in the enclosure can engage with a motor outside the enclosure. For example, the passage in the back wall may allow for a male coupler 530 attached to a motor to engage with a female coupler on the base of the vessel. Male coupler 530 is also shown in FIG. 2 as viewed through a window of system 200.

The system of the disclosure may further comprise a utility cabinet mounted to the safety enclosure. FIG. 3A illustrates utility cabinet 380 mounted to safety enclosure 290. The utility cabinet is also shown in the top and side views of the system in FIGs. 4A and 4B, respectively, and in FIG. 2. The utility cabinet may contain one or more devices or other components included in the system, and can provide for convenient storage and access to those devices or other components without opening the safety enclosure.

The utility cabinet may contain, for example, the means for rotating the vessel such as a motor. As shown in FIG. 5, the motor may engage with the vessel through a passage formed in a wall separating the utility cabinet from the safety enclosure.

The system of the disclosure may further comprise an oxygen concentrator, in fluid communication with the ozone generator, configured to concentrate oxygen from ambient air. The oxygen concentrator, ozone generator or both may be positioned, for example, within the utility cabinet. The system may further comprise a microcontroller, a graphical user interface, or both, optionally positioned within the utility cabinet. FIG. 3A illustrates one possible position for the user interface 390 on the utility cabinet. As with the safety enclosure, the utility cabinet may also comprise windows, such as window 395C, to allow visibility into the interior of the cabinet from the outside.

The system of the disclosure can further comprise any additional devices or components. For example, the system may comprise a particle filter positioned downstream of the gas outlet of the vessel to capture particulate material that may be entrained in gas exiting the vessel. The system may also comprise a transmitter positioned to contact gas withdrawn through the outlet, wherein the transmitter is configured to measure and transmit the concentration of ozone in the gas. An exemplary transmitter is a Model F12 chemical sensor/transmitter available from Analytical Technologies, Inc. One or both of these components may be included within the utility cabinet.

FIG. 7 is a P&ID schematic of a system of the disclosure 700. The system includes a safety enclosure U2 and utility cabinet U1. Safety enclosure U2 comprises a door that is securely closed using magnetic door lock U11. Ambient air is filtered through air filter F1 and dried by air dryer U3. The dried air then enters air compressor U4 then passes through oxygen concentrator U5. Oxygen concentrator U5 may include, for example, a cooler. The gas then enters ozone generator U6. The gas comprising ozone enters vessel U7 that can be rotated about its axis with power provided by AC or DC drive motor M1. One or more motor-driven fans M2 provide negative pressure within the safety enclosure U2.

Gas exiting the vessel is routed through air filter F2 to remove particulates in the exit gas. An optional check valve could be positioned in utility cabinet U1 between F2 and U8. The filtered air passes through buffer tank U8, and ozone concentration transmitter C1 measures the concentration of ozone in the gas. The exit gas then passes through ozone scrubber U9 before being released. Ozone leak detector C2 is included in the utility cabinet to detect ozone gas. Lastly, U10 represents the microcontroller and GUI (graphical user interface) for the system.

The extent to which a contaminant in plant material is reduced as a result of treating it according to the disclosure can be assessed by comparing the amount of contaminant in the plant material before treatment to the amount of contaminant in the plant material after treatment. In some embodiments, the level of a contaminant in the plant material, before and/or after treatment, is measured by analysis in a laboratory. In other embodiments, the system includes an in-line sensor that can detect the amount of contaminant in the material before and/or after treatment, or that can measure the relative reduction of a contaminant in the treated material compared to the untreated material. FIG. 7 illustrates one possible location of such a contaminant sensor C3. The contaminant sensor may be used to measure the amount of any appropriate contaminant, such as bacteria spores or mold, including Aspergillus. In some exemplary contaminant sensors, the sensors may acquire data then calculate or estimate the amount of contaminant by comparing the acquired data to pre-existing reference information for specific contaminants.

A method for treating harvested plant material, which comprises tumbling the harvested plant material within a rotating vessel while exposing the material to ozone, wherein the plant material comprises at least a portion of a cannabis plant, could include, for example:
placing harvested plant material in the vessel of a system of the disclosure as described herein;
generating ozone with the ozone generator;
providing ozone to the vessel from the ozone generator; and
rotating the vessel to tumble the harvested plant material in the presence of the ozone.

The vessel can be filled with any appropriate amount of harvested plant material. In some embodiments, up to one-quarter of the interior volume of the vessel is filled with the material. Additional embodiments include filling up to one-half or up to three-quarters of the interior volume of the vessel with the material. Further embodiments include filling one-quarter or more, or one-half or more, or three-quarters or more of the interior volume of the vessel with the material.

The method of the invention comprises exposing the harvested plant material to any appropriate concentration of ozone, such as ozone at a concentration of 50 ppm to 1000 ppm. The concentration of ozone to which the harvested plant material is exposed may be directly measured within the interior of the vessel or may be estimated based on process inputs and outputs. For example, the concentration of ozone in the vessel may be estimated based on a measured concentration of ozone introduced into an inlet of the vessel (or at a location otherwise upstream of the vessel) or on a measured concentration of ozone at an outlet of the vessel (or at a location otherwise downstream of the vessel).

Some embodiments of the invention include exposing the harvested plant material to ozone at a concentration of 50 ppm to 400 ppm, 50 ppm to 300 ppm, 150 to 300, 200 ppm to 225 ppm, 200 ppm to 300 ppm, 200 ppm to 400 ppm, 100 ppm to 300 ppm, 150 ppm to 250 ppm, or 180 ppm to 220 ppm. In some embodiments, the concentration of ozone to which the plant material is exposed is 50 ppm or greater, 100 ppm or greater, 125 ppm or greater, 150 ppm or greater, 175 ppm or greater, 200 ppm or greater, 225 ppm or greater, 250 ppm or greater, 275 ppm or greater, 300 ppm or greater, 350 ppm or greater, 400 ppm or greater, 450 ppm or greater, 500 ppm or greater, 600 ppm or greater, 700 ppm or greater, 800 ppm or greater, 900 ppm or greater, or 1000 ppm or greater. In some embodiments, the concentration of ozone to which the plant material is exposed is 1000 ppm or less, 900 ppm or less, 800 ppm or less, 700 ppm or less, 600 ppm or less, 500 ppm or less, 400 ppm or less, 350 ppm or less, 300 ppm or less, 275 ppm or less, 250 ppm or less, 225 ppm or less, or 200 ppm or less.

The method of the invention may comprise treating the harvested plant material for any appropriate period of time, such as for a time of from 1 minute to 48 hours.

Some embodiments of the invention include treating the harvested plant material for a time period of 2 minutes to 24 hours, 20 minutes to 18 hours, 20 minutes to 2 hours, 30 minutes to 12 hours, 45 minutes to 6 hours, 1 hour to 4 hours, 1 hour to 2 hours, 1 hour to 12 hours, 2 hours to 6 hours, 4 hours to 8 hours, 4 hours to 18 hours, 6 hours to 12 hours, 10 to 24 hours, or 16 hours to 48 hours. In some embodiments, the time plant material is treated is 1 minute or more, 5 minutes or more, 10 minutes or more, 20 minutes or more, 30 minutes or more, 45 minutes or more, 1 hour or more, 2 hours or more, 3 hours or more, 4 hours or more, 5 hours or more, 6 hours or more, 8 hours or more, 10 hours or more, 12 hours or more, 18 hours or more, 24 hours or more, 30 hours or more or 48 hours or more. In some embodiments, the time plant material is treated is 48 hours or less, 24 hours or less, 12 hours or less, 10 hours or less, 8 hours or less, 6 hours or less, 4 hours or less, 3 hours or less, 2 hours or less, 1.5 hours or less, 1 hour or less, or 30 minutes or less.

Some embodiments of the method of the invention comprise continuously tumbling the harvested plant material throughout its exposure to ozone. Alternatively, the method may comprise intermittently tumbling the harvested plant material throughout its exposure to ozone. For example, the method may comprise treating the harvested plant material in two or more sequences, each sequence comprising 1) rotating the vessel to tumble the harvested plant material in the presence of ozone, and 2) setting the vessel in a static position in which the harvested plant material is exposed to ozone.

The vessel may be rotated to any appropriate extent during the tumbling method, including during any of the tumbling sequences reference above. For example, in the case of a rotating drum having a circular cross section, the method may comprise rotating the drum any appropriate number of degrees β illustrated in FIG. 1C, either in a clockwise or counter-clockwise direction. Thus, embodiments of the method include rotating the drum at an angle β of at least at least 45°, at least 90°, or at least 180°, such as during one or more sequences discussed above.

When the method comprises intermittently tumbling the harvested plant material throughout its exposure to ozone, such as in the sequences discussed above, the method may comprise, for example, setting the drum in a static position for at least 10 seconds, for at least 20 seconds, or for at least 30 seconds, in one or more sequences.

In any embodiments when the drum is rotating, it may be rotated at any appropriate speed, such as at a speed of from less than 1 rpm up to 60 rpm, such as from 1 rpm to 60 rpm, from 5 rpm to 40 rpm, or from 10 rpm to 30 rpm, where rpm is revolutions per minute.

The method of the invention may further comprise generating back pressure in the vessel while treating the harvested plant material. The back pressure could be generated, for example, using a valve to control the flow of gas through an outlet of the vessel and thereby influence gas pressure in the vessel. Back pressure in the vessel can be varied, for example, by opening and closing such a valve to any appropriate extent and frequency during the treatment. Some embodiments of the method include generating back pressure in the vessel of at least 6.9 kPa above atmospheric pressure, or at least 103 kPa above atmospheric pressure, or at least 690 kPa above atmospheric pressure.

Harvested plant material that can be placed in a system used in the method of the invention, or treated in a method of invention, includes any plant material removed from the ground, plant, tree or other host in which it was growing or otherwise present, including seeds of any plant. Exemplary plant materials include a root, stem, stalk, leaf, branch, seed, grain, kernel, sprout, flower or fruit, or any portions thereof, and any other agricultural crop or portion thereof.

As disclosed herein, the plant material can be taken from any species, including microgreens, corn, cork, wheat, tea leaves or soy. The plant material of the inventive method comprises at least a portion of a cannabis plant. The genus of cannabis includes *Cannabis sativa, Cannabis indica,* and *Cannabis ruderalis.* Cannabis includes hemp and marijuana. Cannabis may be used herein to refer to hemp and marijuana or marijuana alone.

Exemplary harvested plant materials include cannabis seeds generally, hemp seeds or marijuana seeds. The term "seeds" refers to material that can be sown to produce a plant. Seed can refer to, for example, an unfertilized plant ovule, a fertilized plant ovule, or an embryonic plant.

According to the inventive method, the harvested plant material comprises at least a portion of a cannabis plant, such as cannabis flower. The harvested plant material includes any such material that is processed or otherwise changed from its natural form after harvesting but before treatment according to the method of the disclosure. For instance, the harvested plant material may comprise homogenized cannabis flower.

Methods of the invention may comprise reducing the amount of one or more contaminants in the harvested plant material. In some embodiments, the methods include reducing the amount of one or more contaminants from one measured level to a lower measured level. Some embodiments also include reducing the amount of one or more contaminants from one measured level to eliminate the contaminant or to reduce the amount of contaminant to a level that is undetectable.

The methods of the invention can be used, for example, to reduce the microbial count of one or more plant pathogens in the material. Microbes or microorganisms that can be included in the microbial count include, for example, microscopic organisms, such as bacteria, fungi, and viruses. In specific, non-limiting examples, microbes include fungi, such as yeast and mold, and bacteria. In some examples, the contamination includes viable aerobic bacteria (TVAB), coliform bacteria, and/or bile-tolerant gram-negative bacteria (BTGN).

In some examples, microbes are present in the untreated harvested plant material at levels that exceed levels allowed for regulatory compliance or are toxic for consumption (such as by inhalation, topical application, or oral delivery), which is also referred to as microbial contamination. Levels of microbes (such as yeast, mold, and bacteria) can be determined in a variety of ways, such as plating and culturing- or quantitative PCT (qPCR)-based techniques (see, e.g., McKeman et al., F1000Res., 5:2471, 2016). Thus, methods of the disclosure can include methods to reduce the amount of fungus (such as yeast, mold or both) in the material, to reduce the amount of bacteria or virus in the material, or to reduce the amount of any combinations of these contaminants in the material.

The acronym "CFU" refers to colony forming units per gram of plant material tested and is unit of measuring microbial levels. Agar plate counting after an incubation period, for example, can be used to measure CFU (such as of yeast, mold, or bacteria). Quantitative Polymerase Chain Reaction, or qPCR, can also be used to measure CFU, by amplifying and detecting a nucleic acid molecule specific for a particular microbe (such as particular genus or species or strain of bacteria, yeast, mold, or virus).

In some embodiments, the initial level of microbial contamination in a plant material, such as cannabis, can be less than 5,000, less than 10,000, less than 50,000, less than 100,000, less than 150,000, less than 200,000, less than 250,000, less than 300,000, less than 350,000, less than 400,000, less than 450,000, less than 500,000, less than 550,000, less than 600,000, less than 650,000, less than 700,000, less than 750,000, less than 800,000, less than 900,000, less than 1,000,000, less than 2,000,000, less than 3,000,000, less than 5,000,000, at least 1,000, at least 5,000, at least 10,000, at least 50,000, at least 100,000, at least 150,000, at least 200,000, at least 250,000, at least 300,000, at least 350,000, at least 400,000, at least 450,000, at least 500,000, at least 550,000, at least 600,000, at least 650,000, at least 700,000, at least 750,000, at least 800,000, at least 900,000, at least 1,000,000, at least 2,000,000, at least 3,000,000, at least 5,000,000, 1,000 to 5,000, 5,000 to 10,000, 10,000 to 50,000, 50,000 to 100,000, 100,000 to 250,000, 250,000 to 500,000, 500,000 to 1,000,000, 1,000,000 to 5,000,000 colony-forming units (CFU) of yeast, mold, virus, and/or bacteria.

The methods can be used to reduce microbial contamination to, for example, less than 150,000 CFUs, less than 100,000 CFUs, less than 50,000 CFUs, less than 40,000 CFUs, less than 30,000 CFUs, less than 20,000 CFUs, less than 10,000 CFUs, less than 9,000 CFUs, less than 8,000 CFUs, less than 7,000 CFUs, less than 6,000 CFUs, less than 5,000 CFUs, less than 4,000 CFUs, less than 3,000 CFUs, less than 2,000 CFUs, less than 1,000 CFUs, less than 500 CFUs, or to no measurable CFUs.

In some embodiments, the method of the disclosure can result in a reduction of microbial contamination (such as yeast, mold, virus, and/or bacteria) in the treated plant material, such as cannabis, by at least 50%, at least 60%, at least 70%, at least 75%, at least 80%, at least 75%, at least 80%, at least 85%, at least 90%, at least 95%, at least 97%, at least 98%, at least 99%, or 100%, or by 50% to 100%, 60% to 100%, 70% to 100%, 75% to 100%, 80% to 100%, 85% to 100%, 90% to 100%, 95% to 100%, 97% to 100%, 98% to 100%, or 99% to 100%, or by 75%, 80%, 85%, 90%, 91%, 92%, 93%, or 94%, 95%, 96%, 97%, 98%, or 99%, or 100% relative to the level of microbial contamination of the plant material prior to treatment.

Methods of the invention may also be used to reduce the amount of one or more pesticides in the material, either alone or together with other contaminants. Non-limiting examples of pesticides include Myclobutanil, Bifenazate, Spiromesifen, and Imidacloprid. For example, pre-treatment plant material such as cannabis can include a level of pesticide greater than or equal to 1 ppb, greater than or equal to 2 ppb, greater than or equal to 5 ppb, greater than or equal to 10 ppb, greater than or equal to 15 ppb, or greater than or equal to 20 ppb.

After treatment, the pesticide in the plant material such as cannabis plant can be reduced, such as by at least 10%, at least 20%, at least 30%, at least 40%, at least 45%, at least 48%, at least 50%, at least 55%, at least 60%, at least 65%, at least 70%, at least 75%, at least 80%, at least 85%, at least 90%, at least 95%, or 100% to a level allowable level for compliance with a regulatory agency or company, such as 1 ppb or less, 2 ppb or less, 5 ppb or less, 10 ppb or less, 15 ppb or less, or 20 ppb or less.

Methods of the invention may be used to treat material to reach compliance with local, state or federal regulations or with standards set by non-governmental companies or institutions. For example, the plant material can have a level of bacteria, yeast, viruses and/or mold that exceeds the allowable level for compliance with a regulatory agency or company prior to treatment, and, following treatment, the pathogen contamination level of the plant material level is reduced to a compliant level. In further embodiments, the plant material has a level of one or more pesticides that exceeds an allowable level for compliance with a regulatory agency or company, and, following treatment, the pesticide level of the plant material level is reduced to a compliant level.

With respect to the treatment of cannabis, the value of cannabis and cannabis-based products to consumers is dependent upon maintenance of the levels of biologically active compounds including THC, CBD and terpenes. For example, many consumers prize the distinct smells produced by aromatic plants, so it can be desirable that the plants maintain those aromas even after being subjected to ozone treatment. Potency is also related to the level of THC. A method useful to treat cannabis and cannabis-based products, such as to ensure compliance with regulatory requirements, would therefore ideally result in the level of THC, THCa, CBD, CBDa, and terpenes remaining substantially the same. In many embodiments, treatment of cannabis plant material significantly reduces or eliminates microbial contamination and/or pesticides in the cannabis plant material, while maintaining substantially the same THC, CBD, and/or terpene content that is important to consumers.

The systems related to the methods of the invention can include a variety of elements in the systems, such as:
one or more processors; and
a memory coupled to the one or more processors, the memory storing instructions that when executed by the one or more processors cause the one or more processors to:
   determine a concentration of ozone at a location within the system;
   adjust the concentration of ozone at the location in the system to a preset concentration such as in the range of 50 ppm to 1000 ppm;
   monitor the concentration of ozone at the location in the system and automatically adjust the monitored concentration to the preset concentration for a period of time such as from 1 minute to 48 hours; and
   rotate the vessel or set the vessel to a static position.
The location in the system may be, for example, within the vessel, at the inlet or outlet of the vessel, or at any other location upstream or downstream of the vessel. The preset concentration of ozone at the location may be, for example, 50 ppm or greater, 100 ppm or greater, 125 ppm or greater, 150 ppm or greater, 175 ppm or greater, 200 ppm or greater, 225 ppm or greater, 250 ppm or greater, 275 ppm or greater, 300 ppm or greater, 350 ppm or greater, 400 ppm or greater, 450 ppm or greater, 500 ppm or greater, 600 ppm or greater, 700 ppm or greater, 800 ppm or greater, 900 ppm or greater, or 1000 ppm or greater.

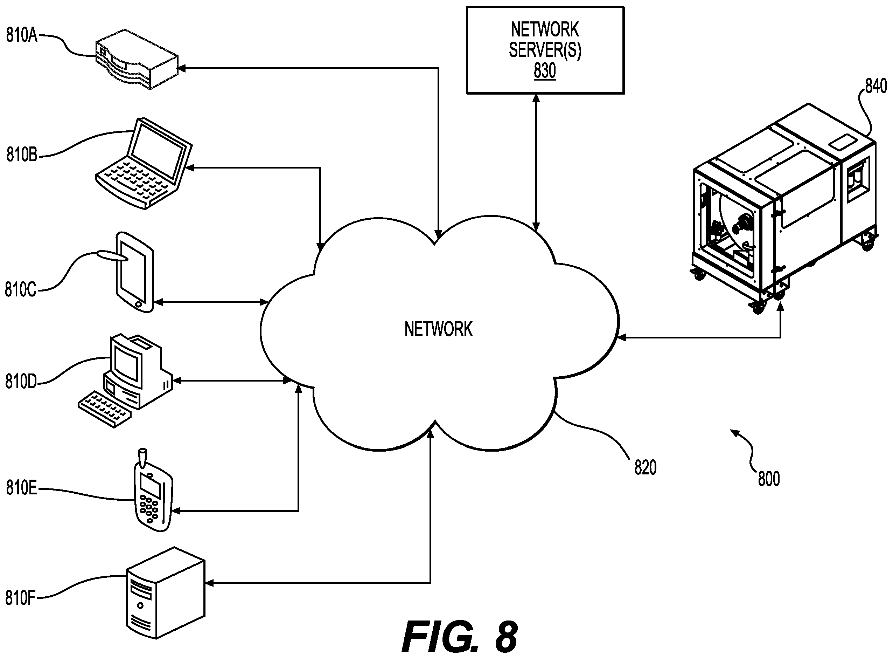

In an exemplary embodiment, FIG. 8 shows a simplified diagram of a distributed computing system 800 in which aspects of the present disclosure may be practiced. Any of computing devices 810A (a modem), 810B (a laptop computer), 810C (a tablet), 810D (a personal computer), 810E (a smart phone), and 810F (a server) may be used to send, receive and evaluate signals from device 840 via one or more network servers 830 and a network 820. A CPU on the device could also be used to send, receive and evaluate such signals. The signals may include data related to ozone concentration and time of exposure, for example. In some embodiments, the system is controlled locally (no network) but monitored remotely over a network, such as a network as just described.

Device 840 may be stationary or mobile. For example, device 840 may stand on a plurality of wheels for moving the device from one place to another. The wheels may be fixed to the device or they may be readily removed and put back on, by for example, a pop out mechanism.

The system of the disclosure may further comprise safety mechanisms including, but not limited to, a destructor for venting gaseous ozone, providing a mechanism for immediately degrading ozone back to oxygen gas, a leak sensor in communicative contact with an alarm display and a safety interlock. One or more of these safety mechanisms may be employed as part of device 840 as well as distributed computing system 800.

A controller (including a "microcontroller" as mentioned herein) may control and operate at least one or all components within the system. The controller may comprise one or more processors and a memory coupled to the one or more processors. The memory may store instructions that when executed by the one or more processors cause the one or more processors to implement one or more steps, including: determining or estimating a concentration of gaseous ozone at a location in the system and adjusting the concentration of gaseous ozone at that location.

The controller may also include a graphical user interface for touch screen operation and system interaction. Integrated sensors may be configured to monitor conditions in the device 840 so that proper action can be taken to reduce pathogen levels associated with plant material being treated. For example, integrated sensors may provide, via a graphical user interface on the pathogen reduction device or a graphical user interface on computing devices 810A-F, an indication that an ozone leak has occurred. The controller may be further configured to shut down one or more of the elements described in the methods and systems described herein to protect the various components of the pathogen reduction device 840. The controller may also be configured to send a signal to one or more of computing devices 810A-F if a sensor has failed such that remedial action can be taken.

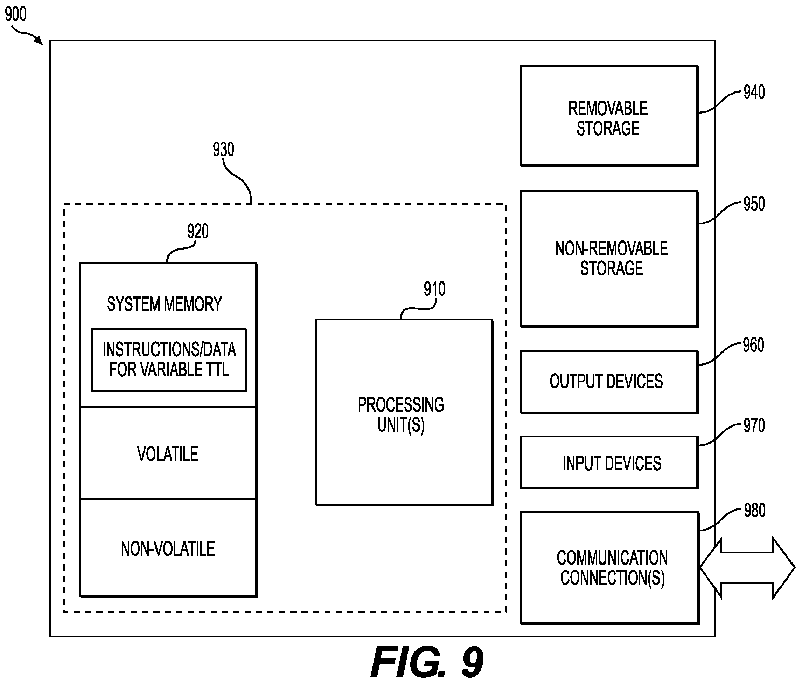

FIG. 9 illustrates one exemplary embodiment of a suitable operating environment 900 in which one or more of the present embodiments may be implemented. FIG. 9 provides only one example of a suitable operating environment and is not intended to suggest any limitation as to the scope of use or functionality. Other well-known computing systems, environments, and/or configurations that may be suitable for use include, but are not limited to, personal computers, server computers, hand-held or laptop devices, multiprocessor systems, microprocessor-based systems, programmable consumer electronics such as smart phones, network PCs, minicomputers, mainframe computers, distributed computing environments that include any of the above systems or devices, and the like.

In its most basic configuration, operating environment 900 typically includes at least one processing unit 910 and memory 920. Depending on the exact configuration and type of computing device, memory 920 (storing, among other things, reputation information, category information, cached entries, instructions to perform the methods disclosed herein, etc.) may be volatile (such as RAM), nonvolatile (such as ROM, flash memory, etc.), or some combination of the two. This most basic configuration is illustrated in FIG. 9 by dashed line 930. Further, environment 900 may also include storage devices (removable, 940, and/or non-removable, 950) including, but not limited to, magnetic or optical disks or tape. Similarly, environment 900 may also have input device(s) 970 such as keyboard, mouse, pen, voice input, etc. and/or output device(s) 960 such as a display, speakers, printer, etc. Also included in the environment may be one or more communication connections, 980, such as LAN, WAN, point to point, etc.

Operating environment 900 typically includes at least some form of computer readable media. Computer readable media can be any available media that can be accessed by processing unit 910 or other devices comprising the operating environment.

By way of example, and not limitation, computer readable media may comprise computer storage media and communication media. Computer storage media includes volatile and nonvolatile, removable and non-removable media implemented in any method or technology for storage of information such as computer readable instructions, data structures, program modules or other data. Computer storage media includes, RAM, ROM, EEPROM, flash memory or other memory technology, CD-ROM, digital versatile disks (DVD) or other optical storage, magnetic cassettes, magnetic tape, magnetic disk storage or other magnetic storage devices, or any other tangible medium which can be used to store the desired information. Computer storage media does not include communication media.

Communication media embodies computer readable instructions, data structures, program modules, or other data in a modulated data signal such as a carrier wave or other transport mechanism and includes any information delivery media. The term "modulated data signal" means a signal that has one or more of its characteristics set or changed in such a manner as to encode information in the signal. By way of example, and not limitation, communication media includes wired media such as a wired network or direct-wired connection, and wireless media such as acoustic, RF, infrared and other wireless media. Combinations of the any of the above should also be included within the scope of computer readable media.

The operating environment 900 may be a single computer operating in a networked environment using logical connections to one or more remote computers. The remote computer may be a personal computer, a server, a router, a network PC, a peer device or other common network node, and typically includes many or all of the elements described above as well as others not so mentioned. The logical connections may include any method supported by available communications media. Such networking environments are commonplace in offices, enterprise-wide computer networks, intranets and the Internet.

Aspects described herein may be employed using software, hardware, or a combination of software and hardware to implement and perform the systems and methods disclosed herein. Although specific devices have been recited throughout the disclosure as performing specific functions, one of skill in the art will appreciate that these devices are provided for illustrative purposes, and other devices may be employed to perform the functionality disclosed herein.

### Example 1

Cannabis is loaded into a 170L aluminum drum which is sealed with an aluminum lid. Three ⅜" polyurethane tubes are connected to the lid via a KF40 flange and rotary bearing mounted to the center of the lid. The three tubes include an ozone inlet, ozone outlet, and a capped service port. A fitting consisting of three ¼" FNPT (inlet) and a KF40 flange (outlet) are used to adapt the three polyurethane tubes to the lid fixture. The base of the drum is fitted with a centered, female coupler.

The drum sits horizontally on a cart containing two rollers parallel to the drum to support the drum weight as shown in FIG. 2. The cart and drum are then wheeled into an enclosure containing a base slotted to fit the cart. As the drum enters the enclosure, the female coupler at the base of the drum makes contact with a male coupler at the back wall of the enclosure. Large radii on the female and male couplers and rotation of the drum allow the two coupler pieces to align so the cart can be fully inserted into the enclosure. The front door of the enclosure is then closed and latched, pressing against a bracket and third roller mounted on the cart, perpendicular to the drum as shown in FIG. 3B. This bracket and roller lock the cart and drum in place as shown in FIG. 3A. The separate cart and enclosure assemblies serve to make loading the drum onto the rollers and loading the drum into the enclosure two separate steps for increased ease of use.

The male coupler fitting is mounted to the shaft of a high torque, single-phase AC or DC motor with a max rotational speed of 11 rpm. The enclosure is fitted with three windows to allow the user to monitor the process/rotation of the drum. The enclosure acts as a safety barrier between the user and moving objects, and as a negative pressure secondary containment chamber for the process. The rear wall of the enclosure is fitted with two high-flow fans blowing out of the chamber to maintain negative pressure (gauge) within the enclosure. The negative pressure ensures that ozone leaking from the drum will not leak into the surrounding environment through the enclosure windows or door. The high-flow fans are mounted to a box filled with Carulite 200 catalyst media (Carus Corporation, Peru, IL), as shown in FIG. 6, to ensure gas is being scrubbed of ozone prior to being pushed into the surrounding environment. A differential pressure switch could be installed in the safety enclosure to ensure that pressure within the enclosure is maintained below the ambient pressure during operation.

A utility cabinet is mounted behind the safety enclosure and contains the drum drive motor, an air compressor, a corona discharge ozone generator, an ozone leak detector, and a microcontroller/GUI system. The air compressor is used to feed gas to the ozone generator at a flowrate of 45L/min. The generator creates a mixture of ozone and the feed gas, which could optionally be fed into a 2L chamber. The chamber acts as a buffer chamber to dampen the pressure pulsation generated by the air compressor. The buffer chamber can be filled with water to create a bubbler, saturating the gas leaving the chamber. To account for this option, a check valve can be installed between the chamber and ozone generator to prevent backflow of water to the generator and a 40µm sintered stainless steel filter can be installed at the inlet of the chamber to diffuse the gas entering the water and ensure full saturation.

The gas exiting the ozone generator, or optional buffer chamber, flows into the process drum containing the cannabis and then out the outlet port. Downstream of the outlet port, the gas flows through a stainless steel 40µm mesh filter. The filter catches any small cannabis particulate to protect downstream components. The filtered gas flows into a second buffer chamber containing an electrochemical ozone sensor and transmitter and then through a Carulite scrubber. An optional valve may be installed between the filter and second buffer chamber to generate back pressure in the drum and increase the activity of the ozone.

During processing (Running State), ozone concentration within the drum is controlled using an ozone transmitter as feedback to a control loop processed by the microcontroller. The output signal of the control loop is power sent to the ozone generator pulsed at a calculated duty cycle. Rotation of the motor is controlled by the microcontroller and is set to repeatedly rotate the drum 180° after 10s of no movement (i.e. the following sequence is maintained through the duration of the run: drum rotates 180°, sits still for 10s, rotates 180°). The static (non-rotating) period of the rotation sequence acts as an exposure period where the top layer of cannabis within the drum is exposed to the gas. The rotation of the drum is used to repeatedly mix the cannabis within the drum to ensure all of the contained cannabis is exposed to an equal amount of ozone through the duration of the run.

This processing sequence is repeated for the process duration specified by the operator. When the specified process time is reached, the system enters the Destructing State, where no power is sent to the ozone generator, allowing compressed air (without ozone) to cycle through the process drum. The rotation sequence used in the Running State is maintained in this state. The ozone concentration leaving the process drum is monitored via the ozone transmitter and the system will remain in the Destructing State until the transmitter reads 0 ppm, indicating that no ozone remains in the drum. The tool then enters the Aerating State, where compressed air continues to be delivered to the process drum while the drum rotates at 11RPM (i.e. no pause in the rotation). This state is used to further destruct any residual ozone remaining on the cannabis and to free any material (e.g. flower) that has stuck to the wall of the drum. Power to the compressor and drum drive motor is then shut off and a notification on the GUI alerts the operator that the material in the tool has been processed (Run Complete State).

Total combined yeast and mold counts (TYMC, or CFU/g) are measured before (control) and after ozone treatment in a system of the disclosure using the processing conditions shown in Table 1. The samples tested were cannabis samples having a weight of about 4.54 kg and volume of about 68L. The ozone gas was at 100% relative humidity by bubbling it through water. Ozone treatment with the system of the disclosure showed a significant reduction in TYMC (CFU/g) compared to untreated controls.

**Table 1**

| **Sample name** | **Process** | **TYMC (CFU/g)** |
|---|---|---|
| 042120-01 | Control (unprocessed) | 8,914 |
| 042220-01 | 250 ppm ozone, 180 minutes | 985 |
| 051120-04 | Control (unprocessed) | 81,984 |
| 051120-01 | 250 ppm ozone, 30 min | 54,488 |
| 051120-02 | 250 ppm ozone, 90 min | 34,836 |

### Example 2

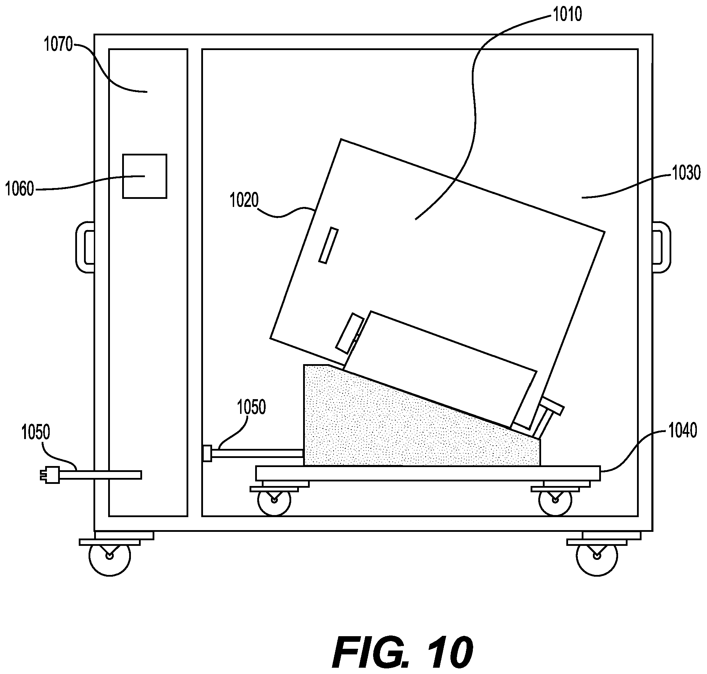

A system of the disclosure is configured such that the vessel is positioned inside an enclosed volume of ozone gas. Such an enclosed volume of ozone gas may be provided by an ozone chamber of a system as described in US 2020/0008428. When placed within such an ozone chamber, the vessel need not include a lid, so that the interior volume of the drum is exposed to the ozone in the ozone chamber. FIG. 10 illustrates such a system. A cylindrical vessel 1010 is placed inside the ozone chamber 1030 of a system described in US 2020/0008428, with open end 1020 of the vessel exposed to ozone gas in the chamber.

The vessel is filled with a harvested plant material, such as cannabis (including, for example, cannabis flower) to a specific fill line, ensuring the vessel is not overfilled and the product cannot fall out. Once the vessel is filled with product, the vessel cart 1040 is placed inside of the chamber. The cart includes 4 wheels, a rotating mechanism, start and stop switch and a plug 1050. The cart is placed on the floor, inside the ozone chamber. The power plug 1050 is wired through the back cabinet 1070 of the system so that it can be plugged into an outlet. The back cabinet may comprise devices and components for ozone generation and measurement and any control mechanisms.

The vessel is placed on top of the cart at a 45-degree angle so the product does not fall out since it will be without a lid. The vessel faces the back end of the chamber where the ozone gas is emitted, ensuring full saturation. The main door to the chamber is then closed to begin in the process. The system is manually turned on from the touchscreen 1060 (HMI, human machine interface) on the outside of the chamber and the vessel rotation is manually turned on with the on/off switch in the back cabinet of the chamber. The vessel can slowly start to rotate and tumble material inside of the vessel at as the material is continually exposed to ozone gas during the treatment time.

The treatment of harvested plant material as defined in the claims, comprising cannabis material, such as cannabis flower, can include, for example, exposing the plant material to ozone concentrations of from 100 to 400 ppm and/or a treatment time of from 1 hour to 12 hours. In some embodiments, the ozone concentration is from 100 ppm to 200 ppm, or from 200 ppm to 300 ppm, or from 300 ppm to 400 ppm. In some embodiments, the treatment time is from 1 hour to 2 hours, 2 hours to 3 hours, 3 hours to 4 hours, 4 hours to 5 hours, 5 hours to 6 hours, 6 hours to 7 hours, 7 hours to 8 hours, 8 hours to 9 hours, 9 hours to 10 hours, 10 hours to 11 hours, 11 hours to 12 hours, 1 to 3 hours, 3 to 6 hours, 6 to 9 hours, or 9 to 12 hours.

When then the treatment/cycle is over, the system will start to destruct all of the ozone within the chamber, converting it back to oxygen. While this is taking place, the vessel can continue to rotate. Once the ozone sensors have determined there is no longer ozone remaining in the chamber, the system will shut off and unlock the chamber door. The vessel will manually be turned off and will slow to a stop. The chamber door will be opened and the vessel will be removed from the chamber. Treated plant material will be moved into a container for storage. The vessel will be cleaned for use on another batch.

### Example 3

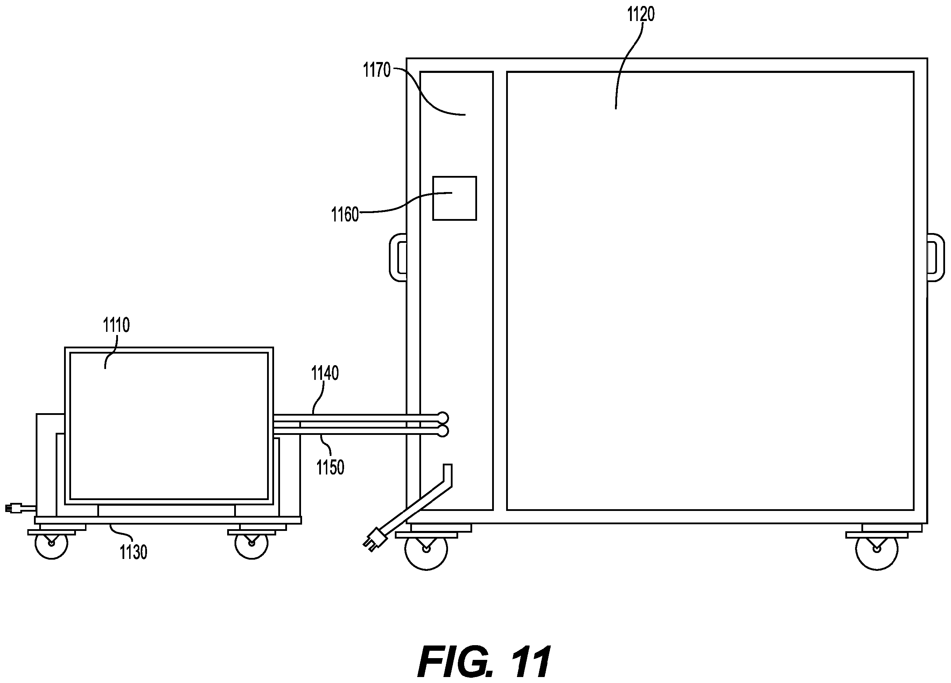

A system of the disclosure is configured such that the vessel is placed within the vicinity of a separate enclosed volume of ozone gas. Such an enclosed volume of ozone gas may be provided by an ozone chamber of a system as described in US 2020/0008428. The vessel may include a lid so as to limit exposure of the interior volume of the drum to its surrounding environment. FIG. 11 illustrates such a system. The cylindrical vessel 1110 is an ozone chamber placed next to and plumbed into a separate existing ozone chamber 1120, to expose the surface area of harvested plant material in the vessel (such as one comprising cannabis flower) to ozone gas.

The vessel is placed on a cart 1130 that includes 4 wheels, a rotating mechanism, start and stop switch and a plug. The vessel is a round chamber with a lid. The plant material is placed in the chamber to a specific fill line. The lid is then be placed on the vessel. The vessel is placed on the stand and is plugged into an outlet.

The system includes tubing 1140 that connects the ozone chamber to an inlet on the backside of the mixing vessel. There is also tubing 1150 to the mixing chamber that will be the destruct media. Both lines of tubing can be plugged into the inlets that then allow ozone gas to flood into the vessel during the treatment time and then pulls out all of the ozone gas at the end of the treatment to destruct back to oxygen. When the tubing lines are plugged into the mixing vessel and fully secure, the system is manually turned on. This will allow ozone gas to start flooding into the mixing vessel. Once ozone production has started, the vessel rotation can be manually turned on, such as from a touchscreen 1160 (HMI, human machine interface) on the outside of the chamber. A back cabinet 1170 may comprise devices and components for ozone generation and measurement and any control mechanisms.

The vessel can continuously slowly turn for the desired treatment time, while the ozone chamber produces ozone gas that is provided into the vessel at the required ozone concentration. Ozone concentrations can be, for example, between 100-400 ppm and treatment times can be, for example, between 1 hours and 12 hours.

When the treatment time has completed, the vessel rotation can be manually turned to off. Then the ozone generated can be manually turned off. The lid of the vessel can remain in place for 1 hour to ensure there is no residual ozone. This time will ensure that all ozone was destructed through the destructing tubes that plumb back into the remainder of the system. Once this hour has been completed, the tubing lines can be removed from the vessel chamber and stored. The vessel lid can then be removed, and product can be taken out of the mixing vessel and placed in a container for storage.

### Example 4

Various strains of cannabis were treated with ozone using a method of the invention. Results of the treatment are summarized in Table 2.

**Table 2**

| | | | | **Terpene (Total %by wt)** | | | | **Potency (Total THC % wt)** | | | |
|---|---|---|---|---|---|---|---|---|---|---|---|
| **Strain Name** | **CFU/g Before** | **CFU/g After** | **Runtime (hrs)** | **Control** | **2hr** | **4hr** | **8hr** | **Control** | **2hr** | **4hr** | **8hr** |
| | | | | | | | | | | | |
| Willow Trim | 8,914 | 985 with 100%RH; 983 dry | 3 | | | | | | | | |
| MiraFlora Mix | 24,347 | 10,880 with 100%RH | 3 | | | | | | | | |
| Jet Fuel | 30,000 | 1,100 | 4 | | | | | 24.1%% | 23.92% | 27.46% | 23.77% |
| Mako Haze | 250,000 | 5,600 | 4 | 1.59% | 1.55% | 1.58% | | 21.17% | 21.15% | 20.12% | |
| Mako Haze | 13,000 | 3,700 | 2 | | | | | | | | |
| Trill OG | 12,000 | 2200 | 4 | | | | | | | | |

| | | | | | | | | | | | |
|---|---|---|---|---|---|---|---|---|---|---|---|
| (RH = relative humidity) | | | | | | | | | | | |

### Example 5

This example illustrates the improved shelf-life of cannabis samples treated according to the disclosure compared to untreated samples. Control samples of untreated cannabis plant material were evaluated for contaminant levels (TYM= total yeast and mold) and other components or properties (TAC= total active compounds) at Day 0 (zero). Samples of cannabis material treated according to the disclosure ("Willow") were similarly evaluated at Day 0. The control samples and treated samples were evaluated again 30 and 60 days later. Table 3 presents the results of these tests.

**Table 3**

| | | 50 = ND | ND= non-detect | | | | | |
|---|---|---|---|---|---|---|---|---|
| **Day 0** | | | | | | | | |
| | **1st Control** | **2nd Control** | **3rd Control** | **Control Avg** | **1st Willow** | **2nd Willow** | **3rd Willow** | **Willow Avg** |
| **TYM** | 1,900 | 50 | 50 | 667 | 370 | 1,100 | 50 | 507 |
| **TAC** | 7,300 | 130,000 | 1,900 | 46,400 | 44,000 | 13,000 | 230 | 19,077 |
| **Potency** | 18.93 | 19.20 | 19.92 | 19.35 | 19.76 | 22.26 | 20.23 | 20.75 |
| **Terpenes** | 2.43 | 2.34 | 2.64 | 2.47 | 2.67 | 3.01 | 3.19 | 2.96 |
| **Water Activity** | 0.32 | 0.35 | 0.33 | 0.33 | 0.30 | 0.33 | 0.26 | 0.30 |
| | | | | | | | | |

| **Day 30** | | | | | | | | |
|---|---|---|---|---|---|---|---|---|
| | **1st Control** | **2nd Control** | **3rd Control** | **Control Avg** | **1st Willow** | **2nd Willow** | **3rd Willow** | **Willow Avg** |
| **TYM** | 9,800 | 380 | 4,500 | 4,893 | 50 | 750 | 1,500 | 767 |
| **TAC** | 31,000 | 2,900 | 45,000 | 26,300 | 100 | 750 | 6,500 | 2,450 |
| **Potency** | 21.04 | 27.74 | 19.00 | 22.59 | 16.53 | 17.63 | 20.65 | 18.27 |
| **Terpenes** | 1.630 | 1.563 | 1.346 | 1.51 | 1.034 | 1.175 | 1.456 | 1.22 |
| **Water Activity** | 0.32 | 0.31 | 0.57 | 0.40 | 0.41 | 0.357 | 0.33 | 0.36 |
| | | | | | | | | |

| **Day 60** | | | | | | | | |
|---|---|---|---|---|---|---|---|---|
| | **1st Control** | **2nd Control** | **3rd Control** | **Control Avg** | **1st Willow** | **2nd Willow** | **3rd Willow** | **Willow Avg** |
| **TYM** | 50 | 5,300 | 1,900 | 2,417 | 1,200 | 380 | 750 | 777 |
| **TAC** | 9,600 | 36,450 | 7,650 | 17,900 | 1,500 | 2,010 | 6,600 | 3,370 |
| **Potency** | 20.43 | 19.36 | 19.68 | 19.82 | 20.98 | 20.01 | 18.77 | 19.92 |
| **Terpenes** | 1.724 | 1.510 | 1.940 | 1.72 | 2.300 | 2.292 | 1.658 | 2.08 |
| **Water Activity** | 0.22 | 0.22 | 0.22 | 0.22 | 0.23 | 0.238 | 0.32 | 0.26 |

The treated Willow samples exhibit an enhanced shelf life compared to the control samples. For example, the average TYM for the treated samples increased about 51% (from 507 to 767) in 30 days and about 53% (from 507 to 777) in 60 days. In contrast, the average TYM for the control samples increased at least about 262% (from 667 to 2,417) in 60 days. Treatment of harvested plant material according to the method of the invention is therefore believed to hinder the growth of bacteria and mold, providing the treated material with a longer shelf life.

## Claims

1. A method for treating harvested plant material, which comprises tumbling the harvested plant material within a rotating vessel while exposing the material to ozone, wherein the plant material comprises at least a portion of a cannabis plant.

2. The method of claim 1 for treating harvested plant material, which comprises:
placing harvested plant material in the vessel of a system, wherein the system comprises:
an ozone generator; and
a vessel in fluid communication with the ozone generator;
wherein the vessel comprises an interior volume for containing harvested plant material and is configured to rotate about an axis, and
wherein the vessel comprises an inlet for gas to enter the vessel and an outlet for gas to exit the vessel, which further comprises a transmitter positioned to contact gas withdrawn through the outlet, wherein the transmitter is configured to measure and transmit the concentration of ozone in the gas;
generating ozone with the ozone generator;
providing ozone to the vessel from the ozone generator; and
rotating the vessel to tumble the harvested plant material in the presence of the ozone.

3. The method of claim 1 or claim 2, which comprises treating the harvested plant material in the presence of ozone at a concentration of 100 ppm to 400 ppm for a time of 1 hour to 12 hours.

4. The method of any one of claims 1-3, which comprises continuously tumbling the harvested plant material throughout its exposure to ozone.

5. The method of any one of claims 1-3, which comprises intermittently tumbling the harvested plant material throughout its exposure to ozone.

6. The method of claim 5, which comprises treating the harvested plant material in two or more sequences, each sequence comprising 1) rotating the vessel to tumble the harvested plant material in the presence of ozone, and 2) setting the vessel in a static position in which the harvested plant material is exposed to ozone.

7. The method of any one of claims 1-6, which comprises generating back pressure in the vessel while treating the harvested plant material.

8. The method of claim 7, wherein the system comprises a valve to control the flow of gas through an outlet of the vessel and thereby influence gas pressure in the vessel.

9. The method of claim 8, which comprises adjusting the valve to vary the back pressure in the vessel during treatment of the harvested plant material.

10. The method of any one of claims 1-9, wherein treating the harvested plant material comprises reducing the microbial count of one or more plant pathogens in the material.

## Patentansprüche

1. Verfahren zum Behandeln von geerntetem Pflanzenmaterial, wobei das Verfahren das Umwälzen des geernteten Pflanzenmaterials in einem rotierenden Behälter aufweist, während das Material Ozon ausgesetzt ist, wobei das Pflanzenmaterial mindestens einen Teil einer Cannabispflanze aufweist.

2. Verfahren nach Anspruch 1 zum Behandeln von geerntetem Pflanzenmaterial, aufweisend:
Einbringen von geerntetem Pflanzenmaterial in einen Behälter eines Systems, wobei das System Folgendes aufweist:
einen Ozongenerator; und
ein Behälter, der in Fluidverbindung mit dem Ozongenerator steht;
wobei der Behälter ein Innenvolumen zur Aufnahme von geerntetem Pflanzenmaterial aufweist und so eingerichtet ist, dass er sich um eine Achse dreht, und
wobei der Behälter einen Einlass, über den Gas in den Behälter eintritt, und einen Auslass, über den Gas aus dem Behälter austritt, aufweist, und ferner einen Sender aufweist, der so angeordnet ist, dass er mit dem Gas in Kontakt kommt, das über den Auslass austritt, wobei der Sender so eingerichtet ist, dass er die Ozonkonzentration in dem Gas misst und überträgt;
Erzeugen von Ozon mit dem Ozongenerator;
Einleiten von Ozon aus dem Ozongenerator in den Behälter; und
Drehen des Behälters, um das geerntete Pflanzenmaterial in Gegenwart des Ozons umzuwälzen.

3. Verfahren nach Anspruch 1 oder Anspruch 2, wobei das Verfahren das Behandeln des geernteten Pflanzenmaterials in Gegenwart von Ozon in einer Konzentration von 100 ppm bis 400 ppm für eine Zeit von 1 Stunde bis 12 Stunden aufweist.

4. Verfahren nach einem der Ansprüche 1 bis 3, wobei das Verfahren das kontinuierliche Umwälzen des geernteten Pflanzenmaterials während der gesamten Ozonexposition aufweist.

5. Verfahren nach einem der Ansprüche 1 bis 3, wobei das Verfahren das intermittierende Umwälzen des geernteten Pflanzenmaterials während der gesamten Ozonexposition aufweist.

6. Verfahren nach Anspruch 5, wobei das Verfahren das Behandeln des geernteten Pflanzenmaterials in zwei oder mehr Sequenzen aufweist, wobei jede Sequenz Folgendes aufweist: 1) Drehen des Behälters, um das geerntete Pflanzenmaterial in Gegenwart von Ozon umzuwälzen, und 2) Platzieren des Behälters in einer statischen Position, in der das geerntete Pflanzenmaterial Ozon ausgesetzt wird.

7. Verfahren nach einem der Ansprüche 1 bis 6, wobei das Verfahren das Erzeugen von Gegendruck in dem Behälter während der Behandlung des geernteten Pflanzenmaterials aufweist.

8. Verfahren nach Anspruch 7, wobei das System ein Ventil aufweist, um den Gasstrom durch einen Auslass des Behälters zu steuern und dadurch den Gasdruck in dem Behälter zu beeinflussen.

9. Verfahren nach Anspruch 8, wobei das Verfahren das Einstellen des Ventils aufweist, um den Gegendruck in dem Behälter während der Behandlung des geernteten Pflanzenmaterials zu variieren.

10. Verfahren nach einem der Ansprüche 1 bis 9, wobei das Behandeln des geernteten Pflanzenmaterials das Reduzieren der mikrobiellen Anzahl von einem oder mehreren Pflanzenpathogenen in dem Material aufweist.

## Revendications

1. Procédé de traitement de matériau végétal récolté, qui comprend le culbutage du matériau végétal récolté au sein d'une cuve rotative tout en exposant le matériau à l'ozone, dans lequel le matériau végétal comprend au moins une portion d'une plante de cannabis.

2. Procédé selon la revendication 1 pour le traitement du matériau végétal récolté, qui comprend :
le placement de matériau végétal récolté dans la cuve d'un système, dans lequel le système comprend :
un générateur d'ozone ; et
une cuve en communication fluidique avec le générateur d'ozone ;
dans lequel la cuve comprend un volume intérieur destiné à contenir le matériau végétal récolté et est configurée pour tourner autour d'un axe, et
dans lequel la cuve comprend une entrée pour le gaz devant entrer dans la cuve et une sortie pour le gaz devant sortir de la cuve, qui comprend en outre un transmetteur positionné de façon à entrer en contact avec le gaz retiré par la sortie, dans lequel le transmetteur est configuré pour mesurer et transmettre la concentration d'ozone dans le gaz ;
la génération d'ozone avec le générateur d'ozone ;
la fourniture d'ozone à la cuve à partir du générateur d'ozone ; et
la rotation de la cuve pour culbuter le matériau végétal récolté en présence de l'ozone.

3. Procédé selon la revendication 1 ou la revendication 2, qui comprend le traitement du matériau végétal récolté en présence d'ozone à une concentration de 100 ppm à 400 ppm pendant une durée de 1 heure à 12 heures.

4. Procédé selon l'une quelconque des revendications 1 à 3, qui comprend le culbutage continu du matériau végétal récolté pendant toute sa durée d'exposition à l'ozone.

5. Procédé selon l'une quelconque des revendications 1 à 3, qui comprend le culbutage par intermittence du matériau végétal récolté pendant toute sa durée d'exposition à l'ozone.

6. Procédé selon la revendication 5, qui comprend le traitement du matériau végétal récolté en deux séquences ou plus, chaque séquence comprenant 1) la rotation de la cuve pour culbuter le matériau végétal récolté en présence d'ozone, et 2) le placement de la cuve dans une position statique dans laquelle le matériau végétal récolté est exposé à l'ozone.

7. Procédé selon l'une quelconque des revendications 1 à 6, qui comprend la génération d'une contre-pression dans la cuve tout en traitant le matériau végétal récolté.

8. Procédé selon la revendication 7, dans lequel le système comprend une vanne pour réguler l'écoulement de gaz à travers une sortie de la cuve et ainsi influencer la pression de gaz dans la cuve.

9. Procédé selon la revendication 8, qui comprend le réglage de la vanne pour faire varier la contre-pression dans la cuve pendant le traitement du matériau végétal récolté.

10. Procédé selon l'une quelconque des revendications 1 à 9, dans lequel le traitement du matériau végétal récolté comprend la réduction de la numération microbienne d'un ou plusieurs pathogènes végétaux dans le matériau.
